(19) Europäisches Patentamt
European Patent Office
Office européen des brevets

(11) **EP 2 534 594 B1**

(12) **EUROPEAN PATENT SPECIFICATION**

(45) Date of publication and mention
of the grant of the patent:
**11.04.2018 Bulletin 2018/15**

(21) Application number: **11702842.3**

(22) Date of filing: **11.02.2011**

(51) Int Cl.:
***G06F 19/00*** (2018.01)     *G06F 19/18* (2011.01)

(86) International application number:
**PCT/EP2011/052004**

(87) International publication number:
**WO 2011/098546 (18.08.2011 Gazette 2011/33)**

(54) **COMPUTATION OF THE COMBINED POTENCY OF DRUG REGIMENS FROM FITTED OR IN VITRO DETERMINED DOSE-RESPONSE CURVES**

BERECHNUNG DER KOMBINIERTEN STÄRKE VON ARZNEIMITTELDOSIERUNGEN AUS ANGEPASSTEN ODER IN-VITRO-BESTIMMTEN DOSISREAKTIONSKURVEN

CALCUL DE PUISSANCE COMBINÉE DE DOSAGES DE MÉDICAMENTS À PARTIR DE COURBES DOSE-RÉPONSE DÉTERMINÉES IN VITRO OU AJUSTÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.02.2010 EP 10153501**

(43) Date of publication of application:
**19.12.2012 Bulletin 2012/51**

(73) Proprietor: **Janssen Sciences Ireland UC Little Island, County Cork (IE)**

(72) Inventor: **CEULEMANS, Hugo Karel B-3060 Bertem (BE)**

(74) Representative: **van Wanrooij, Eva Johnson & Johnson Patent Law Department Turnhoutseweg 30 2340 Beerse (BE)**

(56) References cited:
• **SHEN LIN ET AL: "Dose-response curve slope sets class-specific limits on inhibitory potential of anti-HIV drugs", NATURE MEDICINE, NATURE PUBLISHING GROUP, NEW YORK, NY, US LNKD-DOI:10.1038/NM1777, vol. 14, no. 7, 1 July 2008 (2008-07-01), pages 762-766, XP008119741, ISSN: 1078-8956 [retrieved on 2008-06-15] cited in the application**

**Description**

The invention relates to a method for computing the combined instantaneous inhibitory potential (CIIP) of a drug regimen based on predicted or in vitro determined dose-response curves of the individual drugs used in a treatment regimen wherein said CIIP obtained, is a predictor of the clinical outcome.

By the end of 2007, an estimated 33 million people lived with Human Immunodeficiency Virus (HIV), the causative agent of Acquired Immuno-Deficiency Syndrome (AIDS). To this date there is no known cure for this disease, and progress towards a protective vaccine has been slow. Nevertheless, since the discovery of the HIV, 25 anti-retroviral drugs from six drug classes have been approved for clinical use in its treatment. The current treatment of choice for HIV is Highly Active Antiretroviral Therapy (HAART), which refers to the combination of two or more active drugs from two or more drug classes. The key to treatment success is the choice of drugs that together are potent enough to prevent the replication of the specific strain of the virus targeted. Over a number of months, a successful replication block will result in a gradual decline of the viral load to below the clinical detection level of 50 copies/mL of patient plasma. However, if full inhibition of replication is not achieved because of adherence problems, suboptimal pharmacokinetics or a preexisting resistance of the virus to one or more of the selected drugs, the remaining drug pressure will direct any level of ongoing replication along a path of increasing drug resistance and hence replication. This will ultimately lead to viral rebound and co-morbidity.

Because the transmitted or acquired resistance of a given viral strain is of critical importance to therapy success, current treatment guidelines advise phenotypic or genotypic resistance testing to guide the treatment selection for newly diagnosed or rebound HIV patients. Phenotypic resistance tests are *in vitro* assays with hybrid virions that are in part derived from the patient's virus. They determine for each of the drugs considered, the concentration that inhibits the replication of half of the test virions, i.e. the median effect concentration or $IC_{50}$. The test report will either provide the $IC_{50}$ of the patient and of a fully sensitive reference virus, or their ratio, which is typically referred to as a fold change (FC). From a clinical point of view, the main drawbacks of phenotypic tests are the longer turn-around time and higher cost.

Genotypic tests provide a faster and cheaper alternative, because they do not require the construction of hybrid virions. Instead, they determine the sequence of relevant regions to identify resistance-associated mutations. However, scoring combinations of the more than 200 phenotypically relevant mutations in terms of the suitability of a considered drug is less than straightforward. Expert opinion panels have formulated guidelines for this purpose, but by necessity these remain manageably concise and hence less accurate. Further complexity can be accommodated by formalizing expert knowledge in rules-based algorithms like the ones developed by Stanford HIVdb, Rega or ANRS. These expert algorithms map mutation combinations to one of a few resistance categories. Machine learning or statistical approaches including cluster and linear discriminant analysis, decision trees and regression have also been deployed for continuous resistance scores. In particular, virtual phenotyping (described in patent application WO 01/79450 and Vermeiren et al., 2007) couples the technical convenience of a genotype with the biological meaning of a phenotype: it translates mutation profiles to a corresponding fold change in $IC_{50}$ based on linear regression.

The rising number and potency of individual antiretrovirals is gradually redirecting attention from the scoring of individual drugs to that of combination regimens. Indeed, the number of drug combinations to consider is growing rapidly. This has certainly created some needed flexibility for lowering therapy burden, both in terms of pill counts as in terms of adverse or side effects. Nevertheless, a sufficient combined potency must remain the primary selection criterion for an antiretroviral regimen. Unfortunately, estimating the potency of combined regimens is not trivial for the clinician.

Recently, crude rules-of-thumb that specify a minimal number of active drugs or drug classes are increasing being challenged with quantitative methods for the prediction of clinical outcome. These include artificial neural networks [Larder *et al.,* 2007] and a fuzzy relation system trained with a genetic algorithm [Prosperi *et al.,* 2004] that predict the change in viral load associated with a regimen from standard clinical parameters. Viral load changes document the short-term efficacy of a treatment, but only indirectly the correlated clinical outcome on longer term. The effect of complementing genotypic input with phenotypic FCs and genetic barrier and progression scores on the predictive performance of linear discriminant analysis and support vector machines, decision trees, logistic regression and logistic model trees has also been explored [Altmann *et al.,* 2007]. The unconventional definition of treatment success as genotype absence may limit the clinical impact of this study. Statistical learning approaches have also been implemented to predict the broadly accepted clinical criterion for long-term success, i.e. a clinically undetectable viral load at 24 weeks. Random forests and logistic regression with higher order interactions [Altmann *et al.,* 2009; Prosperi *et al.,* 2009] and the combination of regression and predicted clinical detectability at 24 weeks from genotypic and phenotypic input with Area Under the (Receiver Operating) Curve (AUC) performances of between 0.70 and 0.75. These analyses were based on different data sets, which may somewhat encumber direct performance comparison. However, the performance of three rule-based expert systems on the sets proved strikingly similar. This suggests that these methods can be considered as roughly equivalent in terms of predictive performance from genotypic and phenotypic input. Inclusion of additional patient information (demographics and treatment history) can result in a modest further improvement of up to 0.02 in AUC [Prosperi *et al.,* 2009], but ensuring a standardized input of this information in a real-life diagnostic setting may prove challenging.

Notably, all of the quantitative methods described require substantial clinical datasets for training. The accommodation of new drugs or new combinations of drug classes in these predictors hence requires a substantial clinical effort. Furthermore, the statistical or machine learning models involved only intend to optimally reproduce the outcome of interest. Neither the form of the model used nor the parameters are inspired by underlying biological processes. In fact, a model that explicitly emulates these processes holds the promise of being able to predict the outcome without training on clinical data.

A first move in that direction was the introduction of the Instantaneous Inhibitory Potential (IIP) [Shen Lin et al., Nature Medicine, 2008-07-01, pages 762-766]. This metric for the effect of a given drug dose incorporates not only the $IC_{50}$ of the phenotypic dose-response curve (and hence the traditional concept of resistance), but also its maximal slope. The latter has largely been ignored in the scoring functions for drug selections, but has a significant and less strain-specific impact on the potency of a given drug. From the dose-response curve of a drug for a given HIV strain in a single round replication assay, one can compute the IIP for any individual drug at its average of trough plasma concentration.

Interestingly, as emphasized on the 16th Conference on Retroviruses and Opportunistic Infections (CROI2009), the IIP can also be interpreted as the log inhibition of infectious events per virus generation. It was pointed out that an HIV dynamics model such as the Perelson one [Perelson *et al.,* 1997], can estimate from a patient's viral load how many infectious events per viral generation occur and must therefore be inhibited. The offset between the needed and the expected inhibition should then be predictive of the long-term clinical outcome of the treatment.

Importantly, for most if not all clinical HIV strains combination treatment is indicated, and it was unequivocally stated at CROI2009 (February 2009) that "how to compute the total inhibitory potential for drug combinations is currently unknown". In a recent module for continued medical education distributed by Clinical Care Options (*http://www.cliinicaloptions.com/HIV.aspx*, published May 2009) it was stated that "from the rules for combining inhibitory potentials, which have not yet been established, the total inhibitory potential values of various possible drug combination would be computed". Very recently, this was repeated in an opinion letter by Shen *et al.* [2009]: "in future studies, it will be important to understand how to calculate the IIP of drug combinations from the IIPs of individual drugs, ...".

Along the line of the recent developments in the field, statistical or machine learning technologies could be deployed to combine the IIP scores for individual drugs with or without other inputs that have been explored in earlier studies. However, such an approach would lock the elegant biologically inspired models for the individual drugs in an integrated model of a form that is mathematically convenient but hard to fathom biologically. As a consequence, it will be hard to integrate novel future insights or models on adherence, pharmacokinetics and dynamics in an intuitive way. Moreover, the predictor would require substantial clinical training data to construct and to accommodate new drugs or drug class combinations.

It is therefore an object of the current invention to provide a method for computing the Combined Instantaneous Inhibitory Potential (CIIP) of a drug regimen based on predicted or *in vitro* determined dose-response curves of the individual drugs. The CIIP will be a predictor of the clinical outcome if

1) combination treatment is indicated

2) different instances of biomolecule sequences or other biomarkers are associated with a different but measurable or predictable shape of the dose-response curve(s) of one or more of the regimen drugs. This precludes analysis of the drug combination as if it were a single drug. Instead, it necessitates the integration of the relative contributions of the individual drugs, which differ between patients

3) the phenotypic response to the combination treatment can be translated to a (surrogate) clinical endpoint

These three conditions are met in the diagnostic problem of predicting the long-term clinical outcome of antiretroviral therapy in HIV-infected patients. For newly diagnosed or virological rebound patients where treatment initiation is warranted as judged from CD4 counts, viral load and co-morbidities, clinical guidelines advise antiretroviral combination treatment. Phenotypic or genotype-based virtual phenotypic assays are available to quantify inhibition of replication of a given HIV strain by individual antiretrovirals. Finally, the amount of viral replication to inhibit to bring the viral load at the established clinical endpoint, i.e. below clinical detection levels, can be estimated with HIV dynamics models.

The current invention relates to the description of cellular phenotypic assays as a pseudo-catalytic reaction. An HIV replication assay, for instance, is modeled as an autocatalytic reaction. This description guides the selection of the most appropriate of a set of equations developed to determine whether the combined effect of two inhibitors of an enzyme is synergistic, additive or antagonistic [Syracuse KC & Greco WR, 1986]. The current invention also comprises an extension of that equation for the accommodation of more than two drugs, which enables the computation of a Combined Instantaneous Inhibitory Potential for regimens with any given number of drugs.

In the context of combination regimens of antiretroviral drugs to treat HIV, CIIP computation depends exclusively on dose-response curve parameters and the median trough plasma concentration for each of the drugs considered. All of these will also be available or can be determined for future drugs and, consequently, a resulting predictor can be updated without the need for clinical training data. Nevertheless, the predictive performance of the method with regard to longer term clinical outcome prediction approaches that of the latest generation of statistical learning algorithms, which require substantial amounts of clinical data for training. Like the latter methods, it outperforms publicly available expert algorithms.

Even though CIIP computation does not strictly require clinical training data, when these are available, it can take advantage of them to refine the biologically interpretable parameters of the underlying equations for better performance. In this way, performance is further improved.

Finally, CIIP computation can be useful for the clinical validation of novel phenotypic assays or predictors by comparing the performance with and without inclusion of the phenotypic signal. In a similar analysis with statistical or machine learning approaches, the inherent convolution of signal makes it harder to discern the contribution of the phenotypic signal from that of the training data.

For each of the drugs included in a proposed regimen, and for (a) given relevant instance(s) of (a) biomolecular sequence(s) or biomarker(s), a dose-response curve must be provided. A dose-response curve translates drug concentrations (doses) to their phenotypic effects (responses) within a biologically relevant concentration range. For the theragnostic situations that can be addressed with the method of this invention, response values can be interpreted in terms of a (surrogate) clinical endpoint. The dose-response curves can be directly derived from the phenotypic readouts from a cellular assay at increasingly diluted concentrations. Alternatively, they are interpolated by statistical or machine learning methods.

The metric IIP (Instantaneous Inhibitory Potential) has been introduced [Shen *et al.,* 2008] in the context of a single-round HIV replication assay as the drug-induced $\log_{10}$ relative reduction of the number of infectious events per viral generation. However, it can also be taken as the drug-induced $\log_{10}$ relative reduction of other cellular assay events.

Given a drug concentration C, and a fraction of unaffected assay events $f_u$,

$$IIP(C) = -\log_{10}(f_u)$$

For the most common case of dose-response curves that are described as a two-parameter logistic function defined by the median effect concentration $IC_{50}$ and maximal slope $m$, $f_u$ can be formalized as

$$f_u = \frac{1}{1 + \left(\frac{C}{IC_{50}}\right)^m}$$

and hence,

$$IIP(C) = \log_{10}\left(1 + \left(\frac{C}{IC_{50}}\right)^m\right)$$

For clarity CIIP is also formulated for logistic dose-response curves. However, the formula, like that for IIP, can be generalized for use with all other algebraically defined monotonic functions.

Rather than computing the combined IIP of a regimen indirectly as a function of the IIP scores of the individual drugs, it can calculated directly from the dose-response curves of the component drugs. To this end, the cellular phenotypic assay was modeled as a pseudo-catalytic reaction. Thus, receptor assays can be considered as second-order catalytic reactions and infection assays, such as HIV replication assays can be described as an autocatalytic reaction. In a biochemical assay with an enzyme as catalyst, and under the assumption of additivity, the fraction of enzyme $f_u$ that is unaffected by either of two inhibitors at concentrations $C_i$ and with dose-response parameters $IC_{50,i}$ and $m_i$, is the root of one of the two following functions, depending on whether or not the two inhibitors bind to the same mutually exclusive site [adapted from Syracuse & Greco, 1986].

For mutually exclusive inhibitors:

$$\mathcal{F}_E(x) = \frac{C_1}{IC_{50,1}} \cdot \left(\frac{x}{1-x}\right)^{\frac{1}{m_1}} + \frac{C_2}{IC_{50,2}} \cdot \left(\frac{x}{1-x}\right)^{\frac{1}{m_2}} - 1$$

For non-exclusive inhibitors, the fraction of double-bound enzyme is represented by an extra term:

$$\mathcal{F}_{NE}(x) = \frac{C_1}{IC_{50,1}} \cdot \left(\frac{x}{1-x}\right)^{\frac{1}{m_1}} + \frac{C_2}{IC_{50,2}} \cdot \left(\frac{x}{1-x}\right)^{\frac{1}{m_2}} + \frac{C_1}{IC_{50,1}} \cdot \frac{C_2}{IC_{50,2}} \cdot \left(\frac{x}{1-x}\right)^{\left(\frac{1}{2m_1}+\frac{1}{2m_2}\right)} - 1$$

Unless $m_1 = m_2$, the unique root $f_u$ of these monotonic functions cannot be solved algebraicly, but can be approached numerically by bisection in $\log_2$ of the desired accuracy iteration steps.

For cellular phenotypic assays, the second of these two functions is the more appropriate, even if the drugs under consideration inhibit the same target enzyme by binding through mutually exclusive sites. First, the time scale of the pseudo-catalytic reaction that represents the assay is typically much larger than that of possible underlying enzymatic reactions. Second, drug binding in the pseudo-catalytic model translates at the assay level to much more than just target interaction and includes transport into the cell, intracellular distribution and occasionally processing. For these two reasons, even enzymatically exclusive inhibitors are expected to behave more like non-exclusive inhibitors at the cellular level.

The original Syracuse-Greco functions have been formulated for drug pairs only, and have to be extended for use with multiple drugs. For this purpose, we have derived the following extended functions.

For mutually exclusive inhibitors, the extended formula contains $n + 1$ terms for $n$ drugs:

$$\mathcal{F}_E(x) = -1 + \sum_{i=1}^{n} \left(\frac{C_i}{IC_{50,i}} \cdot \sqrt[m_i]{\frac{x}{1-x}}\right)$$

For non-exclusive inhibitors, the extended formula contains $2^n$ terms to account for all orders of interaction, and requires some additional notation:

Let $\mathcal{P}(D)$ be the powerset of a set D of n drugs, i.e. the set of all $2^n$ subsets $s_j$ of D,

$$p_j = -1 \text{ for } \{\} \text{ and } p_j = \prod_{k=1}^{|s_j|} \left(\frac{C_k}{IC_{50,k}} \cdot \sqrt[|s_j| \cdot m_k]{\frac{x}{1-x}}\right)$$

for all other subsets $s_j$, then

$$\mathcal{F}_{NE}(x) = \sum_{j=1}^{2^n} \left(p_j\right)$$

As with the original Syracuse-Greco functions, the unique root $f_u$ of the latter monotonic functions can be solved numerically by bisection. The thus computed root $f_u$ corresponds to the fraction of pseudo-catalyst inhibited, and by extension to the fraction of unaffected assay events in the cellular phenotypic assay.

The combined IIP of a regimen of any desired number drugs is then formalized as follows, and can be interpreted in the same way as the original IIP formulation for individual drugs.

$$CIIP(C_1, C_2, \dots) = -\log_{10}(f_u)$$

So in summary above a novel and inventive method is described that computes the combined potency of a drug regimen based on predicted or *in vitro* determined dose-response response curves of the individual drugs in a treatment regimen. This is instrumental in those diagnostic situations where different instances of biomolecule sequences or other biomarkers are associated with a different, but measurable or predictable shape of the dose-response curve of one or more of the regimen drugs, and where the response to the drug can be translated to a (surrogate) clinical endpoint. In the context of combination regimens of antiretroviral drugs to treat HIV, the method depends exclusively on genotype-based models for the sensitivity to the individual drugs and three publicly available parameters for each of the drugs considered. Nevertheless, its performance with regard to longer term clinical outcome prediction approaches that of the latest generation of statistical learning algorithms, which require substantial amounts of clinical data for training. Like the latter methods, it outperforms publicly available expert algorithms. As a predictor of the combined virological efficacy of even extensive drug regimens, the approach has the potential to become an important part in the clinical management of HIV. Finally, because the method does not require clinical training data, it enables the quantification of the added value of phenotypic tests or models in clinical outcome prediction of combined drugs therapy without any risk of contaminating clinical signal contribution.

EXAMPLE 1

Demonstration of predictive performance on unseen HIV Treatment Change Episodes (TCEs)

A Treatment Change Episode is defined as a time interval in a patient's treatment history associated with

- stable administration of a recorded antiretroviral drug regimen composed of between 2 and 6 antiretroviral drugs from the following list

  ○ abacavir (ABC)
  ○ atazanavir (ATV)
  ○ zidovudine (AZT)
  ○ stavudine (D4T)
  ○ didanosine (DDI)
  ○ delavirdine (DLV)
  ○ darunavir (DRV)
  ○ efavirenz (EFV)
  ○ etravirine (ETR)
  ○ fosamprenavir (FPV)
  ○ emtricitabin (FTC)
  ○ indinavir (IDV)
  ○ lopinavir (LPV)
  ○ nevirapine (NVP)
  ○ saquinavir (SQV)
  ○ tenofovir (TDF)
  ○ tipranavir (TPV)

- viral load measurements at interval start (baseline) and at 24 weeks
- baseline viral genotypes of the region of interest for the drugs involved The CIIP method was implemented as follows
- for each drug, the median trough concentration from PK studies was used; one convenient compilation of such studies can be found at http://www.hiv-druginteractions.org
- for each drug, as a surrogate single-round $IC_{50}$, the product of the published $IC_{50}$ of a IIIB reference strain in a single-round replication assay [Shen *et al.*, 2008] was used together with the *virtua*lPhenotype™-LM genotype-based predicted fold change in $IC_{50}$ (patent application WO 01/79540; Vermeiren et al., 2007). As a rationale for this choice it is noted that, unlike the $IC_{50}$ itself, the fold change in $IC_{50}$ in single and multi-round phenotypic assays is essentially identical for most drugs [Van Houtte *et al.,* 2009].
- for each drug, the maximal slope of the IIIB reference strain in a single-round replication assay [Shen *et al.,* 2008] was used as is. In biochemical assays, the maximal slope tends to much more stable than the $IC_{50}$ in mutant versions of enzymes. However, any future genotype-based predictions of maximal slopes can be accommodated easily in the presented method and would be expected to increase performance.
- the $f_u$ for a drug combination with these parameters was computed with the above presented, extended Syracuse-Greco equation for non-exclusive inhibitors and, subsequently, the corresponding (unfitted) CIIP was calculated
- even though CIIP can be computed with the above proposed parameters without the need for clinical outcome data, in a second exercise the impact on performance on unseen data was quantified, of optimization of the initial parameter set in terms of performance on a training set of clinical outcome data. To this end, 500 iterations were used of Nelder-Mead simplex optimization on the inverted initial maximal slopes, scaled to their respective initialization values. These slopes, optimized on the training set, were then used instead of the published IIIB maximal slopes [Shen et al., 2008] to compute a fitted CIIP for an independent evaluation set.

According to the invention with "the three publicly available parameters for each of the drugs" is meant: the median trough concentration, the $IC_{50}$ of a IIIB reference strain in a single-round replication assay and the maximal slope of the IIIB reference strain in a single-round replication assay.
A comparison was made of the predictive performance of untrained and trained CIIP (which are the subjects of this invention), weighted Phenotypic Sensitivity Score (wPSS), a statistical learning method described in detail in european patent application ep 09.164.150.6, and three public expert rule-based algorithms, namely REGA, HIVdb and ANRS, to predict treatment failure or success at 24 weeks. The evaluation set contained 1479 unseen TCEs; and a set of 3831 comparable TCEs were used to train wPSS and fitted CIIP. Success was defined as clinically undetectable viral load at 24 weeks, with missing outcomes classified as failures. The Area Under the (Receiver Operating) Curve (AUC) for the optimal logistic regression model controlling for baseline viral load was used as the performance metric.

Table 1: Comparison of the predictive performance (AUC) of a state-of-the-art statistical learning method wPSS, unfitted and fitted CIIP and three rule-based expert algorithms.

| Scheme | All patients | Naïve patients | Salvage patients |
|---|---|---|---|
| *Evaluation set #* | *1479* | *539* | *447* |
| Fitted CIIP | 0.75 | 0.68 | 0.80 |
| Unfitted CIIP | 0.71 | 0.64 | 0.79 |
| wPSS | 0.70 | 0.62 | 0.80 |
| REGA | 0.64 | 0.61 | 0.72 |
| HIVdb | 0.61 | 0.56 | 0.62 |
| ANRS | 0.60 | 0.56 | 0.63 |

Overall CIIP performance approaches that of the statistical learning wPSS and those reported for other statistical or machine learning methods [Altmann *et al.,* 2009; Prosperi *et al.,* 2009]. Like these methods, CIIP outperforms three well-established rule-based expert algorithms.

EXAMPLE 2

Use as a validation method for computing the contribution of phenotyped information to outcome prediction

Quantification of the added value of any clinical test in treatment choice requires a function that maps the test results to a (surrogate) clinical end point. Such a function enables the computation of prediction performance based on the actual patient-specific test information or, alternatively, using general default value that reflects the absence of information. It is critical that no parameter in the function can be directly or indirectly affected by the clinical data that are predicted, because such influences may unfairly increase the observed added value of the test.

The presented method can be parameterized with three parameters derived from phenotypic assays ($IC_{50}$ and maximal slope) and pharmacokinetics studies (median trough concentration) only. Therefore, it produces outcome predictions that are guaranteed to be uncontaminated by clinical outcome data.

Knowledge of strain-specific fold-changes raises the AUC performance on the above described unseen TCEs from 0.62 to 0.71. Given that an AUC of 0.5 is defined as uninformative, this indicates that drug potency (the shape and general parameterization of the function) accounts for slightly more than half of the predictive power (57% of the total AUC increase over 0.5), and strain-specific drug susceptibility information for the remainder for the set used in this example.

REFERENCES

Altmann A, Beerenwinkel N, Sing T, Savenkov I, Däumer M, Kaiser R, Rhee SY, Fessel WJ & Lengauer T (2007) Improved prediction of response to antiretroviral combination therapy using the genetic barrier to drug resistance. Antivir. Ther. 12:169-178

Altmann A, Sing T, Vermeiren H, Winters B, Van Craenenbroeck E, Van der Borght K, Rhee SY, Shafer R, Schülter E, Kaiser R, Peres Y, Sönnerborg A, Fessel WJ, Incardona F, Zazzi M, Bacheler L, Van Vlijmen H, Lengauer T (2009) Advantages of predicted phenotypes and statistical learning models in inferring virological response to antiretroviral therapy from HIV genotype. Antivir. Ther. 14:273-83

Larder B, Wang D, Revell A, Montaner J, Harrigan R, De Wolf F, Lange J, Wegner S, Ruiz L, Perez-Elias MJ, Emery S, Gatell J, D'Arminio Monforte A, Torti C, Zazzi M & Lande C (2007) The development of artificial neural networks to predict virological response to combination HIV therapy. Antivir. Ther. 12:15-24

Perelson AS, Essunger P, Cao Y, Vesanen M, Hurley A, Saksela K, Markowitz M & Ho DD (1997) Decay characteristics of HIV-1-infected compartments during combination therapy. Nature 387:188-91

Prosperi M, Di Giambenedetto S, Trotta MP, Cingolani A, Ruiz L, Baxter JD, ClevenBergh P, Perno CF, Cauda R, Ulivi G, Antinori A & De Luca A (2004) Antivir. Ther. 9:S121

Prosperi M, Altmann A, Rosen-Zvi M, Aharoni E, Borgulya G, Bazso F, Sönnerborg A, Schülter E, Struck D, Van-Damme AM, Vercauteren J & Zazzi M (2009) Investigation of expert rule bases, logistic regression and non-linear machine learning techniques for predicting response to antiretroviral treatment. Antivir. Ther. 14:433-42

Shen L, Peterson S, Sedaghat A, McMahon MA, Callender M, Zhang H, Zhou Y, Pitt E, Anders KS, Acosta EP, Siliciano RF (2008) Dose-response curve slope sets class-specific limits on inhibitory potential of anti-HIV drugs. Nat. med. 14:762-6

Shen L, Rabi SA, Siliciano RF (2009) A novel method for determining the inhibitory potential of anti-HIV drugs. Trends Pharmacol. Sci. 30:610-6

Syracuse KC & Greco WR (1986) Comparison between the method of Chou and Talalay and a new method for the assessment of the combined effects of drugs: a Monte-Carlo simulation study. Proc. Biopharm. Sect. Am. Stat. Assoc. 127-132

Van Houtte M, Picchio G, Van Der Borght K, Pattery T, Lecocq P & Bacheler LT (2009) A comparison of HIV-1 drug susceptibility as provided by conventional phenotyping and by a phenotype prediction tool based on viral genotype. J. Med. Virol. 81:1702-9

Vermeiren H, Van Craenenbroeck E, Alen P, Bacheler L, Picchio G & Lecocq P (2007) Prediction of HIV-1 drug susceptibility phenotype form the viral genotype using linear regression modeling. J. Virol. Methods 145:47-55

**Claims**

1. A computer-implemented method for computing the combined instantaneous inhibitory potential (CIIP) of a drug regimen based on predicted or in vitro determined dose-response curves of the individual drugs used in a treatment regimen wherein said CIIP obtained is a predictor of the clinical outcome and wherein said CIIP is determined according to the following calculations wherein C represents a given drug concentration, $f_u$ represents a fraction of unaffected events, $IC_{50}$ represents the median effect concentration, m represents the maximal slope:

   **Ia: For mutually exclusive inhibitors:**

   The extended formula contains n + 1 terms for n drugs:

   $$F_E(x) = -1 + \sum_{i=1}^{n} \left( \frac{C_i}{IC_{50,i}} \cdot \sqrt[m_i]{\frac{x}{1-x}} \right)$$

   **Ib: For non-exclusive inhibitors:**

   Let P (D) be the powerset of a set D of n drugs, i.e. the set of all $2^n$ subsets $s_j$ of D,

   $$p_j = -1 \text{ for } \{\} \text{ and } p_j = \prod_{k=1}^{|s_j|} \left( \frac{C_k}{IC_{50,k}} \cdot \sqrt[|s_j| \cdot m_k]{\frac{x}{1-x}} \right)$$

   for all other subsets $s_j$, then

EP 2 534 594 B1

$$F_{NE}(x) = \sum_{j=1}^{2^n} \left( p_j \right)$$

**II:** Wherein the unique root $f_u$ of the latter monotonic functions Ia) or I$_b$) can be solved numerically by bisection and the thus computed root $f_u$ corresponds to the fraction of pseudo-catalyst inhibited, and by extension to the fraction of unaffected assay events in the cellular phenotypic assay; and

**III:** Wherein then the combined IIP of a regimen of any desired number drugs is formalized according to $CIIP(C_1, C_2, ...) = -\log_{10}(f_u)$.

2. Method for computing CIIP according to claim 1 wherein for said computing genotype-based models for the sensitivity of the individual drugs are used.

3. Method for computing CIIP according to claim 2 where in addition three publicly available parameters for each of the drugs considered are used.

## Patentansprüche

1. Computergestütztes Verfahren zum Berechnen des kombinierten momentanen hemmenden Potenzials (Combined Instantaneous Inhibitory Potential, CIIP) einer Arzneimitteldosierung aufgrund von vorhergesagten oder in-vitro-bestimmten Dosisreaktionskurven des individuellen Arzneimittels, das in einer Behandlungsdosierung verwendet wird, wobei das erhaltene CIIP eine Vorhersage des klinischen Ergebnisses ist, und wobei das CIIP gemäß den folgenden Berechnungen ermittelt wird, wobei C eine gegebene Arzneimittelkonzentration darstellt, $f_u$ einen Bruchteil nicht beeinträchtigter Ereignisse darstellt, $IC_{50}$ die mittlere Wirkstoffkonzentration darstellt, m die maximale Steigung darstellt:

**Ia:** Für sich gegenseitig ausschließende Hemmer:

Die erweiterte Formel enthält n+1 Terme für n Arzneimittel:

$$F_E(x) = -1 + \sum_{i=1}^{n} \left( \frac{C_i}{IC_{50,i}} \cdot \sqrt[m_i]{\frac{x}{1-x}} \right)$$

**Ib:** Für sich nicht ausschließende Hemmer:

Wenn P(D) die Potenzmenge einer Menge D von n Arzneimitteln ist, d. h. die Menge aller $2^n$ Teilmengen $s_j$ von D ist,

$$p_j = -1 \quad \text{für} \quad \{\} \quad \text{und } p_j = \prod_{k=1}^{|s_j|} \left( \frac{C_k}{IC_{50,k}} \cdot \sqrt[|s_j| \cdot m_k]{\frac{x}{1-x}} \right)$$

für alle anderen Teilmengen $s_j$, dann gilt

$$F_{NE}(x) = \sum_{j=1}^{2^n} \left( p_j \right)$$

**II:** wobei die eindeutige Wurzel $f_u$ der letzteren monotonen Funktionen Ia) oder Ib) numerisch durch eine Bisektion und wobei die somit berechnete Wurzel $f_u$ dem Bruchteil eines gehemmten Pseudokatalysators entspricht und durch eine Erweiterung des Bruchteils eines nicht beeinträchtigten Assay-Ereignisses in dem zellulären phänotypischen Assay gelöst werden kann; und

**III:** wobei dann das kombinierte IIP einer Dosierung einer beliebigen Anzahl von Arzneimitteln gemäß $CIIP(C_1, C_2, ...) = -\log_{10}(f_u)$ formalisiert wird.

2. Verfahren zum Berechnen des CIIP nach Anspruch 1, wobei genotypgestützte Modelle für die Empfindlichkeit der individuellen Arzneimittel für das Berechnen verwendet werden.

3. Verfahren zum Berechnen des CIIP nach Anspruch 2, wobei zusätzlich drei öffentlich verfügbare Parameter für jedes der betrachteten Arzneimittel verwendet werden.

**Revendications**

1. Procédé implémenté par ordinateur permettant de calculer le potentiel inhibiteur instantané combiné (CIIP) d'un dosage médicamenteux sur la base de courbes dose-réponse déterminées in vitro ou prédites des médicaments individuels utilisés dans un régime thérapeutique dans lequel ledit CIIP obtenu est un prédicteur du résultat clinique et dans lequel ledit CIIP est déterminé en fonction des calculs suivants dans lesquels C représente une concentration donnée en médicament, $f_u$ représente une fraction d'événements non affectés, $IC_{50}$ représente la concentration inhibitrice médiane, m représente la pente maximale :

**Ia : pour les inhibiteurs réciproquement exclusifs :**

la formule développée contient $n + 1$ termes pour $n$ médicaments :

$$\mathcal{F}_E(x) = -1 + \sum_{i=1}^{n}\left(\frac{c_i}{IC_{50,i}} \cdot \sqrt[m_i]{\frac{x}{1-x}}\right)$$

**Ib : pour les inhibiteurs non exclusifs :**

soit P (D) l'ensemble des parties d'un ensemble D de n médicaments, à savoir l'ensemble de tous les $2^n$ sous-ensembles $s_j$ de D,

$$p_j = -1 \text{ pour } \{\} \text{ et } p_j = \prod_{k=1}^{|s_j|}\left(\frac{C_k}{IC_{50,k}} \cdot \sqrt[|s_j| \cdot m_k]{\frac{x}{1-x}}\right)$$

pour tous les autres sous-ensembles $s_j$ ; alors

$$\mathcal{F}_{NE}(x) = \sum_{j=1}^{2^n}(p_j)$$

**II :** où la racine unique $f_u$ des précédentes fonctions monotones Ia) ou Ib) peut être résolue numériquement par bissection et la racine $f_u$ ainsi calculée correspond à la fraction d'un pseudo catalyseur inhibé, et par extension à la fraction d'événements de dosage non affectés dans le dosage phénotypique cellulaire ; et
**III :** où l'IIP combiné d'un régime d'un nombre quelconque de médicaments est formalisé en fonction de $CIIP(C_1, C_2, ...) = -\log_{10}(f_u)$.

2. Procédé de calcul de CIIP selon la revendication 1 dans lequel, pour ledit calcul, des modèles basés sur le génotype pour la sensibilité des médicaments individuels sont utilisés.

3. Procédé de calcul de CIIP selon la revendication 2 dans lequel, en outre, trois paramètres disponibles publiquement pour chacun des médicaments envisagés sont utilisés.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0179450 A, Vermeiren
- WO 0179540 A, Vermeiren

### Non-patent literature cited in the description

- **SHEN LIN et al.** *Nature Medicine,* 01 July 2008, 762-766
- **ALTMANN A ; BEERENWINKEL N ; SING T ; SAVENKOV I ; DÄUMER M ; KAISER R ; RHEE SY ; FESSEL WJ ; LENGAUER T.** Improved prediction of response to antiretroviral combination therapy using the genetic barrier to drug resistance. *Antivir. Ther.,* 2007, vol. 12, 169-178
- **ALTMANN A ; SING T ; VERMEIREN H ; WINTERS B ; VAN CRAENENBROECK E ; VAN DER BORGHT K ; RHEE SY ; SHAFER R ; SCHÜLTER E ; KAISER R.** Advantages of predicted phenotypes and statistical learning models in inferring virological response to antiretroviral therapy from HIV genotype. *Antivir. Ther.,* 2009, vol. 14, 273-83
- **LARDER B ; WANG D ; REVELL A ; MONTANER J ; HARRIGAN R ; DE WOLF F ; LANGE J ; WEGNER S ; RUIZ L ; PEREZ-ELIAS MJ.** The development of artificial neural networks to predict virological response to combination HIV therapy. *Antivir. Ther.,* 2007, vol. 12, 15-24
- **PERELSON AS ; ESSUNGER P ; CAO Y ; VESANEN M ; HURLEY A ; SAKSELA K ; MARKOWITZ M ; HO DD.** Decay characteristics of HIV-1-infected compartments during combination therapy. *Nature,* 1997, vol. 387, 188-91
- **PROSPERI M ; DI GIAMBENEDETTO S ; TROTTA MP ; CINGOLANI A ; RUIZ L ; BAXTER JD ; CLEVENBERGH P ; PERNO CF ; CAUDA R ; ULIVI G.** *Antivir. Ther.,* 2004, vol. 9, S121
- **PROSPERI M ; ALTMANN A ; ROSEN-ZVI M ; AHARONI E ; BORGULYA G ; BAZSO F ; SÖNNERBORG A ; SCHÜLTER E ; STRUCK D ; VANDAMME AM.** Investigation of expert rule bases, logistic regression and non-linear machine learning techniques for predicting response to antiretroviral treatment. *Antivir. Ther.,* 2009, vol. 14, 433-42
- **SHEN L ; PETERSON S ; SEDAGHAT A ; MCMAHON MA ; CALLENDER M ; ZHANG H ; ZHOU Y ; PITT E ; ANDERS KS ; ACOSTA EP.** Dose-response curve slope sets class-specific limits on inhibitory potential of anti-HIV drugs. *Nat. med.,* 2008, vol. 14, 762-6
- **SHEN L ; RABI SA ; SILICIANO RF.** A novel method for determining the inhibitory potential of anti-HIV drugs. *Trends Pharmacol. Sci.,* 2009, vol. 30, 610-6
- **SYRACUSE KC ; GRECO WR.** Comparison between the method of Chou and Talalay and a new method for the assessment of the combined effects of drugs: a Monte-Carlo simulation study. *Proc. Biopharm. Sect. Am. Stat. Assoc.,* 1986, 127-132
- **VAN HOUTTE M ; PICCHIO G ; VAN DER BORGHT K ; PATTERY T ; LECOCQ P ; BACHELER LT.** A comparison of HIV-1 drug susceptibility as provided by conventional phenotyping and by a phenotype prediction tool based on viral genotype. *J. Med. Virol.,* 2009, vol. 81, 1702-9
- **VERMEIREN H ; VAN CRAENENBROECK E ; ALEN P ; BACHELER L ; PICCHIO G ; LECOCQ P.** Prediction of HIV-1 drug susceptibility phenotype form the viral genotype using linear regression modeling. *J. Virol. Methods,* 2007, vol. 145, 47-55